# DEMANDE DE BREVET EUROPEEN

(11) **EP 0 557 148 A1**
(43) Date de publication de la demande: **25.08.1993**
(21) Numéro de dépôt: 93400224.7
(22) Date de dépôt: 29.01.1993
(51) Int. Cl.: A61K 35/54, A23L 1/305

(54) **Procédé de préparation de compléments alimentaires à base de tissues embryonnaires**

(30) Priorité: 18.02.1992 FR 9201822
(71) Demandeur: Bontemps, Raymond, F-75016 Paris (FR)
(72) Inventeur: Bontemps, Raymond, F-75016 Paris (FR)
(74) Mandataire: Gutmann, Ernest

(57) **Abrégé**

La présente invention concerne un procédé permettant de prélever et de mélanger à des aliments des extraits de substances embryonnaires fraîches renfermant notamment des acides aminés naturels afin d'obtenir un complément alimentaire particulièrement actif.

Selon la figure 1, le principe du procédé est caractérisé par une réserve (1) contenant des substances mésenchymateuses fraîches stockées à basse température qui sont filtrées sur micropore (2) congelées pour être ensuite broyées, diluées dans du sérum physiologique; 4 mixées avec des aliments, elles constituent un complément alimentaire actif.

L'invention est utilisée dans l'industrie alimentaire et la diététique.

## Description

L'invention, objet de ce brevet, concerne un procédé de préparation d'un complément alimentaire obtenu à partir d'un dispositif permettant de prélever des substances mésenchymateuses composées notamment d'acides aminés naturels, substances particulièrement altérables, qui filtrées, congelées, broyées dans une enceinte aseptisée, mélangées à du sérum physiologique serviront ultérieurement à préparer des compléments alimentaires particulièrement actifs.

Les substances mésenchymateuses sont d'origine foetale à caractère biogène et renferment des acides aminés dont les liaisons chimiques sont insaturées. Elles constituent des microsepteurs particulièrement actifs dans la mesure où des liaisons chimiques comportent des radicaux libres qui se fixent facilement sur les tissus des organes vivants. Cette fixation à pour origine des liaisons électriques libres catalysant l'absorption rapide et facilitant une assimilation par des organes malades ou fatigués d'un individu.

En outre, les acides aminés naturels sont particulièrement appréciés pour leurs effets régénérateurs et stimulants. Leur activité est d'autant plus grande que ces substances mésenchymateuses sont fraîchement prélevées et absorbées dans des conditions telles qu'elles ne subissent aucune altération avec le temps. L'utilisation des basses températures, voisines de -20° C permet de maintenir leurs propriétés initiales intactes et de résoudre le problème de conservation.

Jusqu'à présent, on prélevait ces substances mésenchymateuses, d'origine foetale, tel que le liquide allantoïdien, l'extrait ophtalmique, le cartilage de Mechkel et la gelée de Wharton au moyen d'une seringue.

La substance mésenchymateuse biogène recueillie séparément était supposée contenir une certaine proportion de macromolécules du type Acide Ribonucléique (ARN, ADN) génératrices de matières vivantes et d'acides aminés naturels. Ces substances recueillies n'étant pas exemptes de bactéries ou de virus. Elles étaient ensuite dissoutes à la température ordinaire dans une solution de sérum physiologique afin de constituer une gelée. Pour éliminer les traces de bactéries et de virus, on ajoutait des traces d'oxyquinoléine qui jouaient le rôle de conservateur. la gelée était ensuite filtrée pour éliminer les particules dont la grosseur était supérieure à 0,2 µ. Après cette préparation, la gelée était de nouveau diluée dans un volume de sérum physiologique de façon à obtenir une solution dont le taux de protéines ou matières actives analysées au réfractomètre fournissait une valeur de 12 à 15° pour une préparation à base de cartilage de Wechkel et 20° pour une préparation à base de gelée de Wharton.

On obtenait ainsi une substance et une solution qui étaient supposées constituer une des substances fondamentales mésenchymateuses créatrices de tissus natifs vivants.

Cette faculté de régénération s'explique par le fait que les substances mésenchymateuses sont composées de macromolécules non polymérisées, mélangées à des acides aminés natifs dotés d'une mémoire électrique inductive et capable d'être les éléments de base de la régénération des tissus vivants. Elles peuvent s'absorber sélectivement sur certains tissus et constituent des microsepteurs à mémoire.

Il a été également démontré que ces substances mésenchymateuses composées principalement de tissus lâches, natif de nature conjonctive entre les mailles se composaient d'une substance fondamentale, muqueuse à base de protéines constituant des microsepteurs à mémoire.

Suivant l'origine de la substance embryonnaire mésenchymateuse les proportions de tissus conjonctifs natifs, protéines, acides aminés, ou simple molécule d'ADN ou d'ARN varient.

Par exemple, dans le cas de la gelée de Wharton, la substance mésenchymateuse extra embryonnaire représente un magma réticulaire du blastocèle et constitue une substance foetale particulièrement riche en protéines qui tapisse un tissu conjonctif natif malade.

Le cartilage de Wechkel, substance d'origine foetale et mésenchymateuse renferme des fibrilles collogènes ou protéines polymérisées et de très nombreuses fibres élastiques de nature conjonctive applicable en dermatologie.

Lorsqu'on étudie dans le temps l'évolution de ces substances foetales on a constaté qu'elles se transforment soit en fibres musculaires, soit en tissus conjonctifs capables de régénérer la peau ou l'organe spécifique d'un individu. On constate également que ces tissus et fibres secrètent progressivement une circulation sanguine et un réseau électrique nerveux composé de pseudo neurones chargées d'assurer une véritable programmation sensorielle, d'où leur utilisation comme microsepteur.

On a noté également que ces substances mésenchymateuses sont capables de réparer les tissus soumis à une agression particulièrement profonde d'un organe dont le pouvoir régénérateur propre est partiellement détruit. On a ainsi observé que ces substances mésenchymateuses ont le pouvoir de fournir à l'organisme vivant les moyens susceptibles d'assurer sa régénération au moyen d'éléments primaires et polarisés qu'elles contiennent appelés microsepteurs.

Cependant, ces substances mésenchymateuses ne sont particulièrement actives que lorsqu'elles sont fraîches et introduites dans l'organisme dans leur état initial. En outre, la présente de conservateurs détruit l'effet catalytique des éléments essentiels des substances mésenchymateuses. Pour introduire ces substances dans l'organisme dans les meilleurs conditions d'efficacité, on les associe à des extraits d'organes spécifiques.

Pour réaliser ce complément alimentaire, on utilise des moyens :
- de prélèvement in situ dans des conditions aseptiques absolues de substances mésenchymateuses et des extraits d'organes spécifiques (foie, cervelle, etc...),
- de cryogénie et de conservation des substances mésenchymateuses prélevées en l'absence de conservateur,
- une purification par filtration et une dilution dans une enceinte étanche et aseptisée.

La présente invention dénommée : "Procédé de préparation de compléments alimentaires, à base de substances mésenchymateuses, type microsepteurs" caractérisée en ce que le prélèvement est immédiatement opéré dans une enceinte aseptisée à partir d'éléments vivants ou considérés comme tels; ce prélèvement se compose de substances mésenchymateuses renfermant des acides aminés naturels, dans lesquels on ajoute des extraits d'organes d'origine foetale (foie, cervelle, etc...) ou des substances servant à nourrir un foetus (animal ou autre); ce prélèvement est recueilli à une température de -20°C dans un récipient aseptisé où il reçoit un ajout de sérum physiologique de manière à le transformer en magma, ce dernier est filtré sur un micropore afin d'éliminer les particules dont la grosseur est supérieure 0,2 µ éliminant également certaines bactéries et virus; on maintient ensuite ce magma à - 20° C afin de le solidifier, broyé à froid, il sera mélangé à cette température avec du sérum physiologique pour obtenir une solution limpide constituant un complément alimentaire actif.

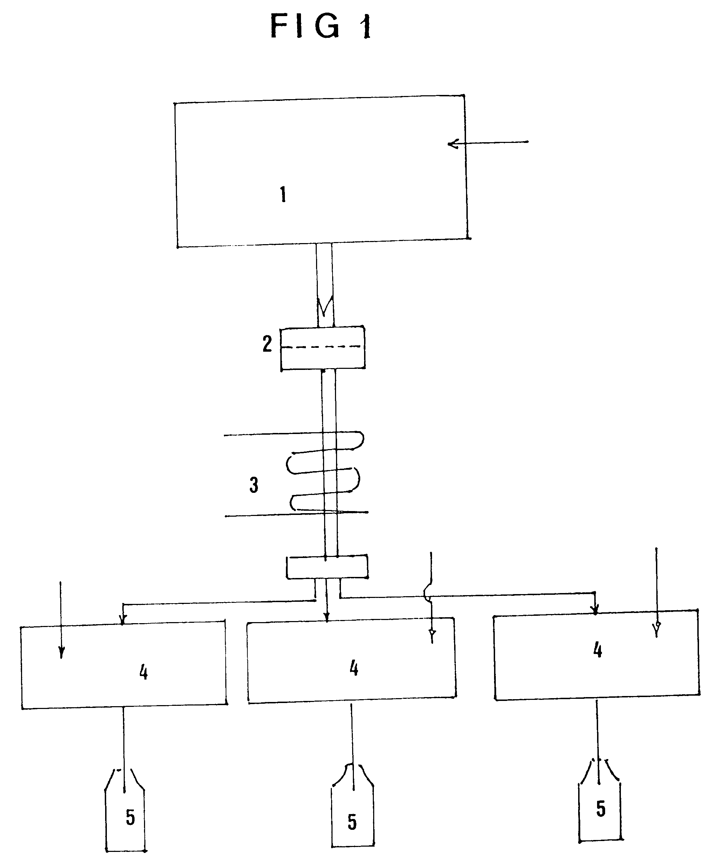

Le procédé de préparation de ces compléments alimentaires sera mieux compris grâce à quelques exemples de fabrications décrits à l'aide de la figure 1 qui est un schéma synoptique représentant les cycles du procédé.

Les prélèvements et la dessication des substances mésenchymateuses tel que l'extrait ophtalmique ou le liquide allantoïdien sont opérés dans les conditions de stérilisation absolue.

Ces différentes substances se trouvent associées aux acides aminés d'origine foetale qui président à la confection d'un foetus.

Selon leur origine (allantoïdien ou ophtalmique) ces substances sont prélevées , mélangées et stockées dans un récipient (1) immédiatement après leur prélèvement. Ce récipient est maintenu à la température de -20° C, condition nécessaire, pour détruire toute prolifération microbienne. On ajoute aux substances mésenchymateuses ainsi recueillies un faible volume de sérum physiologique afin d'obtenir un magma gélatineux. Pour obtenir certaines cellules ou bactéries, cette gelée est filtrée sous pression ou par succion au travers d'un filtre micropore (2) éliminant les particules, et les virus dont la grosseur est supérieure à 0,2 µ. La filtration de ce mélange s'effectue à une température voisine de 0° C au niveau (2) de façon à augmenter la solubilité des substances mésenchymateuses et des extraits d'organes vitaux d'origine foetale (foie, cervelle, etc...) dans le faible volume de sérum physiologique. Immédiatement après la filtration, ce mélange est ramené à une température de - 20° C au niveau (3) au moyen de serpentins de refroidissement.

On obtient au niveau (3), un solide qui peut être broyé à -20° C. Ce broyage permet d'assurer un homogéinisation du mélange et d'obtenir une poudre. Selon l'origine du prélèvement et des extraits d'organes introduits (foie, cervelle) généralement d'origine foetale, associés aux substances mésenchymateuses spécifiques (ophtalmiques, allantoïdien), les poudres obtenues sont traitées dans des circuits distincts placés en parallèles identiques en ce qui concerne le déroulement des étapes du procédé mais qui permettra d'obtenir des compléments alimentaires d'activités différentes et spécifiques.

Ces poudres sont maintenues à -20° C et vont être diluées par un volume de sérum physiologique (4) jusqu'à obtenir une solution limpide que l'on stockera à la température de -20° C dans des flacons (5) étanches dont le volume correspond à une dose utile, et maintenue dans une atmosphère aseptique. Chaque flacon caractérise un traitement de complément alimentaire spécifique à un individu.

## Revendications

**REVENDICATION 1 :** Procédé de préparation de compléments alimentaires à base de substances mésenchymateuses type microsepteurs caractérisé en ce que le prélèvement est immédiat, opéré dans une enceinte aseptisée à partir d'éléments vivants ou considérés comme tels; ce prélèvement se compose de substances mésenchymateuses renfermant des acides aminés naturels d'origine foetales (foie, cervelle, etc...) ou des substances servant à nourrir un foetus (animal ou autre); ce prélèvement est recueilli à une température de -20° C dans un récipient aseptisé où il reçoit un ajout de sérum physiologique de manière à le transformer en magma, ce dernier est filtré sur un micropore afin d'éliminer les particules dont la grosseur est supérieure à 0,2 µ éliminant également certaines bactéries et virus; on maintient ensuite ce magma à -20° C afin de le solidifier, broyé à froid, il sera mélangé à cette température avec du sérum physiologique pour obtenir une solution limpide constituant un complément alimentaire actif placé dans des récipients constituant une dose.

**REVENDICATION 2 :** Procédé de préparation de compléments alimentaires a base de substances mésenchymateuses type microsepteurs selon la revendication 1, caractérisé en ce que le complément alimentaire est spécifique de telle ou telle carence de l'individu au moyen d'un accouplement des substances mésenchymateuses d'origine allantoïdique avec des extraits de foie et' des substances mésenchymateuse d'origine ophtalmique avec des extraits de cervelle ou autres produits nourriciers du foetus afin d'obtenir un complément alimentaire, contenant des acides aminés natifs nécessaires à la régénération sanguine ou nerveuse d'un individu.

**REVENDICATION 3 :** Procédé de préparation de compléments alimentaires à base de substances mésenchymateuses type microsepteurs selon la revendication 1, caractérisé en ce que le mélange substances mésenchymateuses, acides aminés, extraits d'organes tels que foie ou cervelle est spécifique à chaque individu souffrant d'un carence au moyen de microsepteurs polarisés qui sont secrétés entre les mailles de la substance mésenchymateuse chargée d'acides aminés natifs et qui se fixe sur l'organe particulièrement carencé par attraction électrique.

**REVENDICATION 4 :** Procédé de préparation de compléments alimentaires à base de substance mésenchymateuses type microsepteurs selon la revendication 1, caractérisé en ce que le moyen de stocker ces compléments alimentaires fabriqués sans altération sont des récipients contenant une dose de complément alimentaire maintenu à -20° C. (5)

**REVENDICATION 5 :** Procédé de préparation de compléments alimentaires à base de substances mésenchymateuses type microsepteurs selon la revendication 1, caractérisé en ce que le moyen de faire varier l'activité des compléments alimentaires est la dilution commandée dans le mélange par le sérum physiologique sur le complément alimentaire solidifié à -20° C et mis en poudre à cette température.

**REVENDICATION 6 :** Procédé de préparation de compléments alimentaires à base de substances mésenchymateuses type microsepteurs selon la revendication 1, caractérisé en ce que le moyen d'éliminer les virus est une filtration sous pression du mélange substances mésenchymateuses, extraits d'organes et acides aminés sur un micropore dont les orifices de porosité sont inférieurs à 0,2 µ. (2)
